# EUROPEAN PATENT APPLICATION

(11) **EP 3 991 668 A1**
(43) Date of publication of application: **04.05.2022**
(21) Application number: 20204631.4
(22) Date of filing: 29.10.2020
(51) Int. Cl.: A61B 17/34, A61B 5/0538, A61B 5/06, A61B 34/20, A61B 17/00

(54) **DETERMINING PARAMETERS FOR A PENETRATING DEVICE**

(71) Applicant: Koninklijke Philips N.V., 5656 AG Eindhoven (NL)
(72) Inventor: SCHWARTZ, Yitzhack, 5656 AE Eindhoven (NL); IBRAGIMOV, Zalman, 5656 AE Eindhoven (NL)
(74) Representative: Philips Intellectual Property & Standards

(57) **Abstract**

A mechanism for determining one or more parameters of a penetration device and/or surrounding anatomical tissue. The electrical responses of two electrodes mounted on a sheath of the penetration device and a distal end of a needle of the penetration device are monitored, and used to derive the parameters.

## Description

### FIELD OF THE INVENTION

The present invention relates to the field of medical devices, and in particular to devices that penetrate tissue for a desired medical outcome.

### BACKGROUND OF THE INVENTION

There is an increasing interest in the use of penetration devices to perform medical procedures. A penetration device is a device configured to puncture or incise tissue of a patient, e.g. to create a channel between two different anatomical cavities.

By way of example, a process known as a transseptal puncture uses a penetration device to create a channel or hole between the right and left atria. Catheters and delivery systems can be placed through this channel/hole to safely cross the inter-atrial septum (IAS) and be introduced into the left atrium. Accessing the left atrium without the use of a transseptal procedure is a fairly difficult or cumbersome process, as it requires the retrograde manipulation of catheters through the left ventricle and mitral valve.

There is therefore a desire to improve the performance of a penetration process, and in particular, to the performance of a transseptal puncture. Successful performance of a penetration process is directly dependent upon the accuracy, validity and appropriateness of information about the penetration device and/or the surrounding tissue.

It would therefore be advantageous to improve the determination of information about the penetration process, e.g. information about tissue surrounding a penetration device.

Furthermore, transseptal procedures are currently being carried out under fluoroscopy and/or ultrasound (in the case of perforating the IAS, by either trans-esophageal or intra-cardiac echocardiography) guidance. The present disclosure recognizes that a fluoroless solution that can potentially obviate the need to use ultrasound is advantageous.

### SUMMARY OF THE INVENTION

The invention is defined by the claims.

The present disclosure proposes mechanisms and concepts for use of dielectric-based sensing and/or imaging during a penetration process, such as a transsceptal puncture. Proposed approaches can advantageously avoid the need for performing fluoroscopy and/or ultrasound imaging during a penetration process, thereby avoiding the adverse effects of the same on a patient.

In particular, the present disclosure proposes to treat electrically-responsive elements of a penetration device as internal electrodes for detecting electric fields within an anatomical structure. The electric fields are artificially generated electric fields (i.e. not electric fields generated by the biological entity under investigation). The electrical response(s) of the electrically-responsive elements of the penetration device can be used to derive or measure one or more parameters of the penetration device and/or surrounding tissue of the anatomical structure.

According to examples in accordance with an aspect of the invention, there is provided a processing system for determining one or more parameters of a penetration device, comprising a sheath that houses a needle having a distal end for penetrating tissue of an anatomical structure, and/or parameters of surrounding tissue of the anatomical structure.

The processing system comprises: an input interface configured to obtain: at a first input, and from the distal end of the needle, a first set of one or more electrical responses of the distal end of the needle to one or more electrical fields induced in the anatomical structure, wherein the distal end of the needle is formed of a material responsive to changes in an electric field; an at a second input, and from a first electrode mounted on the sheath, a second set of or more electrical responses of the first electrode to one or more electrical fields induced in the anatomical structure; and at a third input, and from a second electrode mounted on the sheath, a third set of one or more electrical responses of the second electrode to one or more electrical fields induced in the anatomical structure; and a processor circuit configured to determine one or more parameters of the penetration device and/or surrounding tissue of the anatomical structure using at least the first set of one or more electrical responses, the second set of one or more electrical responses and the third set of one or more electrical responses.

The present disclosure has recognized that the distal end of a penetration device can advantageously be treated as an electrode for monitoring electrical fields induced in the anatomical structure. This facilitates use of the distal end of the penetrating for determining characteristics of the penetration device and/or the surrounding tissue.

In particular, it is recognized that the combination of electrical responses of a distal end of the needle and two electrodes on a sheath of the penetration device can provide useful information for aiding in the performance of a penetrating process, such as a transseptal procedure. The use of two sheath-based electrodes advantageously allows for tracking of the sheath (and, in particular, a direction in which a needle housed in the sheath faces, even when completely covered by the sheath), whilst using the needle as an electrode allows for the position of the needle to be tracked when it exits the sheath (with the position being definable using the sheath electrode(s)).

For example, the electrical response(s) could be tracked to monitor a location of the distal end and/or the sheath of the penetrating device. This could aid in ensuring that the penetration device is maneuvered to an appropriate location and that appropriate tissue is penetrated.

In a particularly advantageous example, the electrical responses of the electrodes mounted on the sheath could be tracked to perform an imaging process of the anatomical structure, to thereby construct an anatomical model of the anatomical structure. This means that a same device can be used to perform both mapping of the anatomical structure and to perform the penetration, reducing the number of intrusive objects that need to be inserted into the individual, thereby reducing exposure to potential infection or cross-contamination.

As another example, the electrical response(s) could be tracked to derive a thickness of tissue in the vicinity of the distal end, thereby facilitating ease in tracking an appropriate location for penetrating the tissue (e.g. for improved ease in creating a throughhole in the tissue). As yet another example, the electrical response(s) may be processed to identify when the distal end makes contact with tissue, thereby facilitating ease in tracking a penetration process.

Suitable examples of material responsive to changes in an electric field may include medical-grade metals, such as titanium, (surgical) stainless steel, or cobalt-chrome alloy; as well as other suitable materials. The material responsive to changes (for the distal end of the needle) should be sufficiently sturdy to be able to puncture tissue.

The distal end of the needle is designed for puncturing tissue, e.g. formed of a suitably shaped and/or sharp structure for puncturing tissue.

The needle is housed in a sheath, e.g. a largely cylindrical element through which the needle is threaded. The sheath can act to protect tissue surrounding the needle from its distal end until a desired location or zone for performing a penetrating process with the penetration device is reached.

By monitoring one or more parameters of the surrounding tissue and/or the sheath using the electrode mounted on the sheath, additional information for use in a penetration process can be obtained, e.g. the relative location of the sheath during a penetration process. This can facilitate identification of the location of the penetration device, e.g. during transit to the desired location, and/or be used to determine characteristics for surrounding tissue in tandem with the distal end of the needle.

The present disclosure thereby facilitates a mechanism for determining clinically useful characteristics of tissue and/or the penetration device.

The processing system may further comprise an output interface configured to provide, to a further device, an output signal responsive to the determined one or more parameters of the penetration device and/or surrounding tissue of the anatomical structure. The further device may, for example, comprise a user interface, a memory, a further processing system, an alerting system or any other suitable further device for receiving an output signal.

The processing system may be further configured to display, at a user interface, a visual representation of the determined one or more parameters of the penetration device and/or surrounding tissue. Displaying a visual representation of the determined parameters can provide a clinician, e.g. controlling a penetration operation of the penetration device, with useful clinical information to aid their control of the penetration device to achieve a desired medical goal, e.g. penetration or puncturing of tissue. This information can thereby credibly assist a clinician in the performance of a technical task.

The one or more parameters of the penetration device and/or surrounding tissue may comprise a relative position of the sheath and/or the needle with respect to the anatomical structure. Determining a location of the penetration device is clinically helpful in ensuring that the correction penetration location for the tissue has been identified, and to mark (e.g. for subsequent use) a location of the penetration performed by the penetration device. This proposed disclosure thereby facilitates knowledge over a position of a penetration performed by a penetration device.

In some preferred embodiments, the one or more parameters of the penetration device and/or surrounding tissue comprises an orientation/direction of the sheath and/or the needle. This increases an ease of tracking the movement of the needle during the transsceptal procedure, e.g. to ensure that the needle is moving in the correction direction for performing the transsceptal puncture.

In some embodiments, the input interface is further configured to obtain, at a fourth input and from an modelling system or memory, an anatomical model of the anatomical structure, and the one or more parameters of the penetration device and/or surrounding tissue comprises a relative location of the sheath and/or needle with respect to the anatomical model of the anatomical structure.

The skilled person will appreciate that determining a relative location of the penetration device with respect to the anatomical model is effectively equivalent to determining a relative location of the penetration device with respect to the anatomical structure, as the anatomical model is a description of the anatomical structure in encoded form or computer language.

In some examples, the anatomical model of the anatomical structure is generated by monitoring the location of one or more electrodes mounted upon an intraluminal device. In this way, the position of the distal end of the needle can be derived in a same virtual space as used to generate the anatomical model of the anatomical structure. This increases an ease in determining a relative location of the distal end to the anatomical model.

The processing system may be adapted wherein the first or second electrode comprises an anchoring structure configured to secure or couple the sheath to tissue during a penetrating procedure of the penetration device.

Some sheaths for penetration devices may comprise an anchoring structure that connects or anchors the sheath to tissue during a penetration process (e.g. to reduce a movement of the sheath and/or penetration device during the penetration process). The anchoring structure may also be used to apply tension to the tissue to aid in puncturing the tissue with the puncturing device.

The present disclosure recognizes that this anchoring structure could be further repurposed for use as an electrode, and can thereby provide additional information (in the form of electrical responses) for determining one or more parameters of the penetration device. This additional information can be clinically useful. This also reduces the number of electrodes that need to be positioned on the sheath, e.g. if the sheath only comprises the anchoring structure and one other sheath electrode.

In some examples, the anchoring structure is moveable relative to other components of the sheath. The anchoring structure may thereby be used, for example, to brace the sheath against the tissue or to push tissue away from the sheath. The anchoring structure may comprise a loop of material, e.g. a loop of (medical-grade) metal. The movement of the sheath may follow predefined mechanical properties.

There is also proposed a medical system for penetrating tissue of a subject, the medical system comprising: a penetration device comprising: a needle having a distal end for penetrating tissue of an anatomical structure, the distal end of the needle being formed of a first material responsive to changes in an electric field; a sheath that houses the needle; an electrode mounted on the sheath; and the processing system herein disclosed.

The sheath and the needle may be separable or inseparable elements. Thus, the sheath and the needle may form a kit of parts that, when together, form the penetration device.

In some examples, the distal end of the needle is configured to puncture the inter-atrial septum of a subject. In other words, the penetration device may be designed for a transseptal procedure.

The medical system may comprise one or more external electrodes, to be positioned externally to the subject, controllable to generate one or more electric fields, wherein the distal end of the needle is configured to be responsive to changes in the one or more electric fields generated by the one or more external electrodes. In some examples, the processing system may control the operation of the external electrodes, e.g. control an electric field generated by the external electrodes.

There is also proposed a computer-implemented method for determining one or more parameters of a penetration device, comprising a sheath that houses a needle having a distal end for penetrating tissue of an anatomical structure, and/or surrounding tissue of the anatomical structure.

The computer-implemented method comprises: obtaining, from the distal end of the needle, a first set of one or more electrical responses of the distal end of the needle to one or more electrical fields induced in the anatomical structure, wherein the distal end of the needle is formed of a material responsive to changes in an electric field; obtaining, from a first electrode mounted on the sheath, a second set of or more electrical responses of the first electrode to one or more electrical fields induced in the anatomical structure; and determining one or more parameters of the penetration device and/or surrounding tissue of the anatomical structure using at least the first set of one or more electrical responses, the second set of one or more electrical responses and the third set of one or more electrical responses.

The computer-implemented method may further comprise providing, to a further device, an output signal responsive to the determined one or more parameters of the penetration device and/or surrounding tissue of the anatomical structure.

The one or more parameters of the penetration device and/or surrounding tissue may comprise a relative position of the distal end of the needle with respect to the anatomical structure.

There is also proposed a computer program product comprising code which, when executed by a processor circuit, causes the processor circuit to perform the steps of any herein described method. There is also proposed a non-transitory computer-readable medium or data carrier comprising or carrying the computer program product.

The present disclosure also proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

The skilled person would be readily capable of adapting any herein described method to reflect embodiments of herein described apparatus, systems and/or processors, and vice versa. A similar understanding would be made by the skilled person with respect to a computer program (product).

Additional aspects, features, and advantages of the present disclosure will become apparent from the following detailed description.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a penetration device for use in an embodiment;
Fig. 2 illustrates a medical system having a processing system according to an embodiment;
Fig. 3 illustrates a point cloud to surface reconstruction procedure;
Figs. 4 to 6 illustrate a penetrating process;
Figs. 8 and 9 provide a representation of an anatomical model;
Fig. 9 provides a flowchart according to an embodiment; and
Fig. 10 illustrates a processor circuit.

### DETAILED DESCRIPTION OF THE EMBODIMENTS

The invention will be described with reference to the figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the figures are merely schematic and are not drawn to scale. It should also be understood that the same reference numerals are used throughout the figures to indicate the same or similar parts.

The invention provides a mechanism for determining (values of) one or more parameters of a penetration device and/or surrounding anatomical tissue. The electrical responses of two electrodes mounted on a sheath of the penetration device and a distal end of a needle of the penetration device are monitored, and used to derive the parameters.

Embodiments of the invention thereby provide useful information for a clinician to perform a penetrating process, e.g. by tracking the location of important elements of the penetration device. The present invention also recognizes that certain elements of the penetration device can be adapted for use as intra-body or internal electrodes, i.e. can have a dual purpose.

Concepts of the invention can be employed to provide useful clinical information when performing a penetrating process such as a transseptal procedure.

In the context of the present disclosure, the term "internal" refers to within the body of a subject, and the term "external" generally refers to outside of the body of the subject. The term "determine a parameter" means to determine, predict, measure or otherwise define a value or values for a particular parameter.

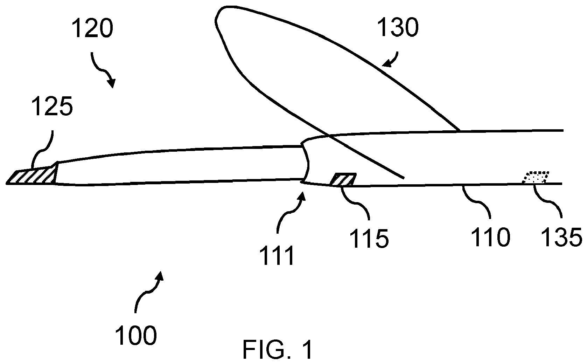

Fig. 1 illustrates a distal end of a penetration device 100 for use in embodiments of the invention. The penetration device 100 comprises a sheath 110, which houses a needle 120. The needle 120 is configured to be movable within the sheath, so that the sheath 120 can cover and uncover the needle as it moves with respect to the sheath 110.

A distal end 125 of the needle 120 is configured or designed for penetrating or puncturing tissue. The needle 120 may, for example, be a transseptal needle for puncturing an intra-atrial septum (IAS). Appropriate transseptal needles would be well known to the skilled person.

The distal end 125 of the needle is configured to be made of a material responsive to changes in an electric field (e.g. in which the needle is placed). In other words, an electrical response of the distal end 125 of the needle 120 may change in response to a change of a parameter (magnitude, frequency etc.) of an electric field into which the needle is positioned. Suitable materials would be apparent to the skilled person and may include, for example, metals (e.g. titanium, (surgical) stainless steel, cobalt-chrome alloy or the like).

In this way, the distal end 125 of the needle 120 can be treated as an electrode, e.g. a unipolar electrode.

A sheath 110 is configured to house the needle 120. Thus, the sheath 110 may comprise a lumen or passage through which the needle 120 can be threaded and moved. The needle 120 may be moveable to be covered and uncovered through a distal end 111 of the sheath 110 (i.e. the needle may be controllably retracted or exposed). The sheath may be formed of a biocompatible material. The sheath may effectively act as a catheter, to thread through one or more body lumens and/or cavities (e.g. to reach the right atrium for performing a transseptal puncture). Thus, the sheath may transport the needle through one or more lumens and/or cavities of the subject.

The penetration device further comprises a first electrode 115 mounted on the sheath 110. The electrical response(s) of the first electrode 115 is configured to be responsive to one or more electric fields into which the first electrode is placed. The first electrode 115 may be an immovable electrode with respect to the sheath, i.e. its relative position on the sheath may be unchanged.

The penetration device 100 further comprises a second electrode 130 mounted on the sheath. Here, the second electrode is embodied as an anchoring structure 130. The anchoring structure 130 is configured to anchor, secure or couple the sheath to tissue (of a patient) during a penetrating procedure, performed using the needle 120. Thus, the anchoring structure 130 may effectively anchor the sheath to the tissue, e.g. to reduce or prevent movement of the sheath during a penetrating procedure or to aid in deflection/bending of the sheath (to guide the needle into an appropriate position).

The anchoring structure 130 is configured to comprise material that is electrically responsive to electric fields into which the anchoring structure is placed. Thus, the anchoring structure can be treated as an electrode, e.g. a unipolar electrode. In particular, as previously explained, the anchoring structure 130 can act as a second electrode mounted on the sheath.

The anchoring structure 130 may, for example, comprise a loop of electrically responsive material, such as the anchoring nitinol loop of the TSP Crosser^{™}transseptal access system (Transseptal Solutions).

Preferably, the anchoring structure 130 may be configured to be controllably movable with respect to the sheath, e.g. to extend out of or retract back into the sheath. This means that the anchoring structure can be deployed (e.g. extended outwardly) as the sheath/needle nears is or at a part of tissue that is to be penetrated/punctured. The illustrated anchoring structure 130 is depicted in an extended position.

In some other embodiments, the second electrode may comprise an electrode 135 which is immovable with respect to the sheath, similar to the first electrode 115. If present, this second electrode 135 is also configured to be responsive to one or more electric fields into which the second electrode is placed. Of course, in such examples, the anchoring structure may still exist, but need not be electrically responsive.

If the anchoring structure 130 is electrically responsive, the electrode 135 may be omitted.

In some embodiments, the penetration device comprises two sheath electrodes, i.e. electrode 115 and electrode 135, and an anchoring structure (i.e. three electrodes mounted on the sheath), as well as the needle. This facilitates yet further improved tracking of components of the penetration device.

From the foregoing, it is apparent that there are at least three possible elements ("electrically-responsive elements") of the penetration device that are responsive to an electric field into which they are placed: the distal end of the needle; the first electrode on the sheath and the second electrode on the sheath, which may be embodied as an anchoring structure for securing the sheath against tissue of the patient.

The present disclosure recognizes that the electrical response(s) of these electrically-responsive elements to electric fields induced in an anatomical cavity can provide useful information. In particular, a processing system (e.g. a dielectric imaging or sensing system) can exploit these responses to generate additional or more accurate information about the penetration device and/or the anatomy of the subject to improve a clinician's understanding.

In particular, the present invention recognizes that these elements are particularly advantageous for use with a penetration device for transsceptal procedures. In particular, this proposed approach allows a same device to be used for both electrical mapping of the anatomical structure (e.g. using the electrodes on the sheath) and for tracking the position and direction of the needle (e.g. using the electrical responses of the needle and one or more of the responses of the sheath electrodes).

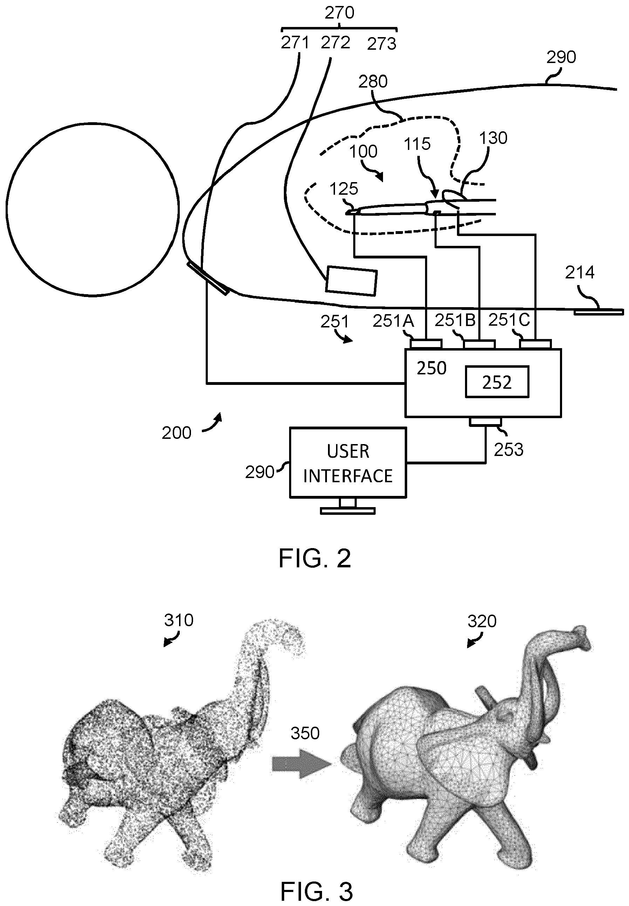

Fig. 2 conceptually illustrates a medical system 200 for penetrating tissue, such as an IAS, of a subject. The medical system 200 comprises the penetration device 100 previously described.

The medical system 200 further comprises a processing system 250 configured to process the response(s) of the previously identified electrically-responsive elements of the penetration device 100 to determine one or more parameters of the penetration device and/or surrounding tissue of the anatomical structure using at least the first set of one or more electrical responses and the second set of one or more electrical responses.

The processing system 250 is, by itself, an embodiment of the invention.

The processing system 250 comprise an input interface 251. The input interface comprises at least a first input 251A, a second input 251B and a third input 251C. The first input 251A is configured to obtain, from the distal end 125 of the needle, a first set of one or more electrical responses to one or more electrical fields induced in the anatomical structure. The second input 251B is configured to obtain, from the first electrode mounted on the sheath, a second set of one or more electrical responses to one or more electrical fields induced in the anatomical structure. The third input 251C is configured to obtain, from the second electrode mounted on the sheath, a third set of one or more electrical responses to one or more electrical fields induced in the anatomical structure.

The input interface 251 of the processing system may therefore be communicatively coupled to the electrically-responsive elements of the penetration device, e.g. using wires position in/on the sheath of the penetration device. Thus, the processing system 250 is configured to electrically communicate with the distal end 125 of the needle and an electrode mounted on the sheath.

Accordingly, the penetration device may comprise one or more wires, each connecting to an electrically-responsive node for connection to the input interface 251 of the processing system 250.

In this way, the processing system may effectively treat at least the distal end 125 of the needle and the electrodes mounted on the sheath ("electrically-responsive elements") as intra-body or internal electrodes.

The processing system comprises a processor circuit 252 that determines one or more parameters of the penetration device and/or surrounding tissue of the anatomical structure based on the first, second and third sets of responses. Different combinations of the first, second and third sets of responses may be used to determine different parameters of the penetration device. Example parameters include a location of the penetration device, an orientation of the penetration device, a shape of the penetration device, a location of the distal end of the needle, a distance that the projection needle extends out of the penetration device, a direction and/or orientation of the needle; a map or model of the anatomical structure, a shape of the anatomical structure, a thickness of tissue of the anatomical structure and so on.

Suitable working examples will be provided later.

The processing system may further comprise an output interface 253 configured to provide, to a further device, an output signal responsive to the determined one or more parameters of the penetration device and/or surrounding tissue of the anatomical structure.

In some examples, the further device is a user interface 290, such as a display. Other suitable examples of a further device include a memory, a further processing system, an alerting system, and so on.

The processing system may be further configured to display, at a user interface, a visual representation of the determined one or more parameters of the penetration device and/or surrounding tissue. Thus, the output signal provided by the output interface may control or define a display provided by the user interface.

In particular examples, each response (of the first, second and third sets) may comprise at least a response to an electric field induced in the anatomical structure by an external electrode.

In some examples, the medical system 200 may further comprise one or more external electrodes 270, to be positioned externally to the subject, controllable to generate one or more electric fields. The electrically-responsive elements of the penetration device respond to these electric fields.

The external electrodes 270 electrodes may comprise a plurality of electrodes angled with respect to one another (e.g. positioned orthogonally to one another), so that any electric fields generated by the electrodes are angled with respect to one another. Preferably there are three pairs of electrodes each pair having electrodes on opposing sides of the subject being investigated such as front and back, neck and lower abdomen and left and right with the region of interest being in between all pairs of electrodes. The external electrodes may also comprise a reference electrode, for example positioned on one of the legs of the subject.

The external electrodes may be controlled to generate electrical fields of different frequencies (and in different directions). The control of the external electrodes may be performed by the processing system, or a separate external controlling device. This results in an internal electrode (i.e. "electrically-responsive element") having a different response to the externally applied electric fields based on its relative position within the subject. This information can be used to track a relative location of the internal electrode within the subject and/or derive other characteristics of the electrode and/or the surrounding tissue.

For instance, if there are three external electrodes 271, 272 273 or pairs of external electrodes positioned to emit electric fields of different frequencies (E₁, E₂, E₃) that are angled (e.g. near-orthogonal) with respect to one another, a voltage response (V₁, V₂, V₃) of an internal (intrabody) electrode (e.g. of a cathter) (e.g. identifying a voltage between the electrode and the reference electrode or between the electrode and the electrode generating the electric field) will differ depending upon position within the anatomical cavity. Other forms of response, such as an impedance response or a capacitive response (e.g. indicating change in impedance/capacitance between the internal electrode and each external electrode) will be apparent to the skilled person. These responses are dependent upon dielectric properties of surrounding tissue and/or the position of the electrode with respect to the electric fields.

Thus, a response to an electric field may comprise a voltage response, an impedance response, a capacitor response and so on. Preferably, the response is a voltage response.

The present disclosure recognizes that the response of electrically-responsive elements of the penetration device to such externally provided electric fields can be processed to provide useful information for aiding the performance of a penetrating process.

It has previously been described how the first, second and third set of electrical responses can be processed by the processor circuit of the processing system to determine one or more parameters of the penetration device and/or surrounding tissue of the anatomical structure. In some examples, this process comprises generating an anatomical model of the anatomical structure using at least the second and third sets of responses (i.e. the responses of the electrodes mounted on the sheath).

Thus, the responses of the electrically-responsive elements of the (sheath of the) penetration device can be used to perform an imaging process of the anatomical structure, thereby generating an anatomical model. The processor circuit of the processing system 250 may therefore process the second and third sets of responses to perform an imaging process. This is particularly advantageous for use with a penetration device, as it allows a single penetration device to perform both mapping/imaging of the anatomical structure and to perform the actual penetration process (e.g. by subsequently tracking the direction/position of a needle).

Use of electrodes mounted on the sheath to perform the imaging process is particularly advantageous, as it allows the needle to be retracted inside the sheath during the imaging process for improved patient safety.

An example imaging process is hereafter described, but it not exclusive for use with electrically-responsive elements of the herein described penetration device. Rather, any suitable internal electrodes could be used when performing a generic imaging process, such as electrodes mounted on a catheter.

To perform an imaging process, the response of the internal electrodes (e.g. to externally applied electric fields) may be iteratively recorded. The processor circuit of the processing system can repeatedly apply a transfer function ("V2R function") that transforms each recorded response to Euclidian coordinates (R-space), whilst ensuring known properties (e.g. electrode spacing and electrical weight length) as well as a set of other constraints are maintained. By way of example only, such a process is disclosed in WO2019/034944 which is incorporated by reference.

In this way, an R-space cloud of points (known Euclidian co-ordinates) can be built up and updated as the internal electrodes are moved within the anatomical cavity. Using the updated R-space cloud of points, a reconstruction algorithm generates an anatomical model of the anatomical cavity. The anatomical model may, for example, be a 3D surface that depicts or models the (bounds of) the anatomical cavity.

The anatomical model may be output to a display or user interface 290. The display or user interface may be configured to provide a visual representation of the anatomical model.

The process of reconstructing an anatomical model from a point cloud is conceptually illustrated in Fig. 3, which demonstrates a process 350 in which a cloud of R-space points 310 ("point cloud") is transformed into an anatomical model 320. In the illustrated example, this is performed by creating a (3D) surface from the point cloud data, methods of which will be readily apparent to the skilled person.

For example, a point cloud can be converted into a polygon mesh or triangular mesh model (or other surface model) using a surface reconstruction approach. A variety of suitable approaches is discussed in Berger, Matthew, et al. "A survey of surface reconstruction from point clouds." Computer Graphics Forum. Vol. 36. No. 1. 2017.

More precise identification of the bounds and features of the anatomical model can be performed by monitoring the response of the electrically-responsive elements of the penetration device (here: treated as internal electrodes) to local field measurements (e.g. fields generated by other internal electrodes) or through additional processing of global field measurements.

For example, regions with inherently marked steep gradients in the electrical field can be identified. It is recognized that such regions indicate the bounds of the anatomical cavity and/or other information, e.g. drainage of vessels into or out of a cardiac chamber as well as the valves of a cardiac chamber. These features are being picked up uniquely by the system and imaged even without physically visiting them with the catheter.

As another example, changes in a local electrical field (between two internal electrodes) can indicate the presence or absence of tissue between the two internal electrodes. A response of an internal electrode to a local electric field can therefore be used to identify the presence or absence of tissue. The processing system may itself be configured to control the local electric fields, or this can be controlled by another device in communication with the electrically-responsive elements of the penetration device.

Such use of local field measurements has been described in e.g. WO WO2019/034944.

The combined global and local field measurements enable sophisticated detection and effective handling of inconsistencies and outliers, level of electrode shielding/coverage (e.g. by measuring location inter-correlation), pacing (saturation), as well as physiological drift. Drift can, for example, be detected using a moving window over time and corrected continuously whereby the catheter location remains accurate throughout the whole procedure making the system resilient to drift.

Other approaches for using external electrodes (e.g. patch electrodes) and internal electrodes (e.g. catheter electrodes) to map body volumes and visualize the locations of catheters within the map can be found in, for example, U.S. Patent No. 10,278,616, titled "Systems and Methods for Tracking an Intrabody Catheter," filed May 12, 2015, and U.S. Patent No. 5,983,126, titled "Catheter Location System and Method," filed August 1, 1997, the entireties of which are hereby incorporated by reference.

In some examples, the responses of internal electrodes (or the anatomical model) may be processed to identify a target anatomical feature for identifying a location to perform a penetrating process. For example, if the penetrating process is a transseptal procedure, the target anatomical feature may be the fossa ovalis (FO) or tissue that shows the presence of a Patent Foramen Ovale. Other suitable target anatomical features will be apparent, depending upon the penetrating process.

In some example, the responses of internal electrodes (or the anatomical model) may be processed to identify and localize areas near target anatomical features, for example, to identify potential danger areas for a penetrating process. As one example, for a transseptal process, the responses (or the anatomical model) may be processed to identify and localize the Aorta behind the IAS. This information can be used by a clinician to avoid inadvertent puncturing of the Aorta while advancing the needle across the IAS.

From the foregoing, it will be apparent that one example of a parameter of the penetration device and/or surrounding tissue of the anatomical structure is an anatomical model of the anatomical structure (i.e. a shape/structure of the surrounding tissue). Another example parameter is a position of a target anatomical feature.

The processing system may be configured to (render and) display, at a user interface, a visual representation of the anatomical model. The visual representation of an anatomical model may, for example, comprise a 3D or flattened (panoramic) view of the anatomical structure.

Another example of a parameter of the penetration device and/or surrounding tissue of the anatomical structure is a (relative) position of the penetration device or particular electrically-responsive elements of the penetration device.

In particular, a relative position of the penetration device with respect to an anatomical model (e.g. generated using the previously described imaging process) could be determined. Once an anatomical model/map of the anatomical structure has been generated by monitoring the response(s) of electrodes about the anatomical structure to electric fields, the relative position of an electrode with respect to the anatomical model/map can be readily determined by correlating a response of the electrode to a particular position.

Thus, embodiments of the present disclosure propose to further use the processing system to track a location of one or more electrodes (of another catheter) with respect to the anatomical model, i.e. to act as an electrode tracking system.

It will be apparent that, once an anatomical model of an anatomical cavity has been constructed using responses of internal electrodes within the anatomical cavity, then the location of internal electrodes with respect to the anatomical model can be readily defined. For example, the transfer function used to generate the anatomical map, e.g. the "V2R function" that transforms a response to Euclidian coordinates, can be used to calculate/predict a relative position of an electrically-responsive element of the penetration device using the response of the internal electrode.

The input interface 251 of the processing system 250 may be further configured to obtain, at a fourth input (not shown) and from a modelling system or memory, an anatomical model of the anatomical structure. The anatomical model of the anatomical structure is preferably generated by monitoring the location of one or more electrodes mounted upon an intraluminal device, e.g. the penetration device or another intraluminal device.

The processor circuit 252 of the processing system 250 may be configured to determine the relative location of one or more electrically-responsive elements of the penetration device with respect to an anatomical model. The relative location of the one or more electrically-responsive elements can be derived from various combination of the first, second and third sets of the responses. The anatomical model may, itself, be generated by monitoring the response of internal electrodes to an electric field, e.g. using the approach previously described.

In particular, the first set of one or more responses may be used to track a location of the distal end of the needle, and the second set of one or more responses may be used to track a location of the sheath or the overall penetration device. The present disclosure recognizes that information on both of these two features can contribute to improved monitoring of the penetrating process.

This process facilitates visualization of the electrically-responsive elements of the penetration device with respect to the anatomical structure.

The processing system may therefore be further configured to display, at a user interface, a visual representation of the relative location of the (electrically-responsive elements of the) penetration device. This can be displayed with respect to a visual representation of the anatomical model, e.g. obtained at the user interface.

Although preferable, when tracking/monitoring the location of electrically-responsive elements of the penetration device with respect to an anatomical model, it is not essential that this anatomical model be generated using the penetration device, rather a different set of internal electrodes (e.g. mounted on a catheter) could be used. Of course, a combination of both approaches could be used, i.e. the penetration device can contribute to the mapping of the anatomical structure.

Tracking the location of electrically-responsive elements of the penetration device advantageously allows a clinician to visualize and perform a full penetrating procedure (e.g. a transseptal process) solely under dielectric sensing & imaging, i.e. without fluoroscopy or ultrasound guidance.

In particular, the maneuvering of the penetration device to a desired location can be performed by tracking the location of the sheath (which can cover the needle to shield other tissue from the needle) and the penetrating process itself can be tracked by tracking the distal end of the needle (e.g. to track the process of the needle penetrating tissue).

Once a suitable location for performing the penetrating process has been identified, the location can be marked (e.g. with respect to the anatomical model). This can aid in future investigation of the area, e.g. for reuse of the penetration in subsequent procedures, or in case penetration is to be performed by another device.

Another example of a parameter of the penetration device and/or surrounding tissue is a distance that the needle extends out of the sheath. Thus, the processor circuit may be configured to process a combination of the first second and third sets of responses to determine a distance that the needle extends out of the sheath

For example, if the relative position of the electrode(s) on the sheath and the relative position of the distal end of the needle is known, then the distance between the electrode(s) and the distal end of the needle can be calculated. This information can be used to derive/measure the distance that the needle extends out of the sheath, e.g. if the relationship between the electrode(s) and the end of the sheath is known.

As another example, the difference in the electrical responses of the electrode(s) mounted on the sheath and the distal end of the needle may be directly correlated to a distance between the electrode(s) and the distal end of the needle and/or a distance that the needle extends out of the sheath.

This information can be used to check whether or not the needle is safely covered by the sheath, e.g. during transport of the needle towards a site for penetrating tissue.

Another example of a parameter of the penetration device and/or surrounding tissue is an orientation of the penetration device. In particular, if the (3D) positions of two parts of the penetration device with respect to an anatomical model are known, and the relationship between these two parts is predetermined (i.e. due to a known structure of the penetration device), then the relative orientation of penetration device with respect to the anatomical model can also be derived.

This information can be used to improve the display of a penetration device by a user interface, e.g. with respect to a displayed anatomical model. Information on the orientation of the penetration device aids a user to understand how the manipulation of the penetration device can be performed, to correctly orient the penetration device during transportation to a penetration location or during the penetration location itself.

In some examples, the orientation information and location information is used to provide a visual representation of the penetration device with respect to a displayed anatomical model (e.g. which is correctly oriented and located within 3D space).

As yet another example, one parameter of the penetration device and/or surrounding tissue may be an orientation/direction of the needle. In particular, the processing system may track the location of the first electrode on the sheath and the needle (using approaches previously described). As the positional relationship between these elements is predetermined, a direction of the needle can be derived (e.g. a direction of the needle may be defined as spanning between the location of the first electrode and the location of the needle).

This information can be used to improve the display of the needle by the user interface, in particular by providing an indication of the direction of the needle, e.g. alongside the displayed anatomical model to facilitate improved performance of the transsceptal procedure. This is achieved as the determined direction aids a clinician in performing the transsceptal procedure more accurately, by providing an indication of the direction orientation of the needle (to ensure that it is penetrating the correct area of the anatomical structure).

It has previously been described how the second electrode mounted on the sheath may be/comprise an anchoring structure 130 mounted on the sheath 110. The processor circuit of the processing system 250 may use the third set of one or more electrical responses of the anchoring structure to electric fields induced in the anatomical structure to derive one or more parameters of the penetration device and/or surrounding tissue of the anatomical structure.

As an example only, the processor circuit of the processing system may use the third set of one or more electrical responses to track a location of the anchoring structure mounted on the sheath. Of course, the tracked location may be displayed via a user interface.

Tracking the location of this anchoring structure also aids in the performance of the penetrating process, as it provides useful information to the clinician. In particular, it can aid a clinician to understand a real-time position of the anchoring structure to thereby appropriately control the brace or support provided to the sheath, through maneuvering the anchoring structure to a particular location for bracing against the tissue, to improve an accuracy in performing a penetrating process.

As the geometric shape of the anchoring structure can be known, it is also possible to display a visual representation of the geometric shape with respect to the determined location of the anchoring structure. For example, the anchoring structure may be shaped as a loop (for bracing the sheath against tissue), which can be displayed with respect to an anatomical model. Displaying the geometric shape of the anchoring structure aids in improving a clinician's understanding of the characteristics of the penetration device and the penetration process.

The anchoring structure may be moveable with respect to the sheath. In particular, the anchoring structure may be able to extend and retract with respect to the sheath. The manner in which the anchoring structure moves may be known, e.g. due to known mechanics of the penetration device. By tracking the location of the anchoring structure (using the third set of responses) with respect to the location of the first electrode on the sheath, the extent to which (i.e. the protruding distance) the anchoring structure extends from the sheath can be determined, as can the shape of the anchoring structure as it extends (due to predetermined, estimated or known device characteristics). This information can be visually represented.

As an example, the anchoring structure may comprise a loop that can be extended out of the sheath. The loop will move accordingly with a preconfigured or known loop's motion. Since the mechanical behavior of the extended loop is known, and the position of the loop is being tracked, the loop itself can also be displayed in real-time.

Previous examples of one or more parameters of penetration device and/or surrounding tissue of the anatomical structure generally rely upon the response of electrically-responsive elements of the penetration device to externally provided electric fields. However, some parameters of the penetration device and/or surrounding tissue of the anatomical structure may be derived from an electrical response of the electrically-responsive elements of the penetration device to internally created electric fields (e.g. electric fields generated by an electrically-responsive element).

In some examples, the processor circuit may be configured to further control one or more electric fields generated by one or more of the electrically-responsive elements of the penetration device. This can be performed by controlling a current supplied to the electrically-responsive element(s), e.g. over a same electric pathway used to measure an electrical response or a dedicated channel. The first, second and third sets of responses may include an electrical response to this internally generated electric field.

Yet another example of a parameter of the penetration device and/or surrounding tissue of the anatomical structure is a measure of contact or contact force between the penetration device and the surrounding tissue of the anatomical structure. This can be performed, for example, using the approach described in the European Patent Application having publication number EP 3294174 A1, with the electrically-responsive elements of the penetration device being treated as electrodes.

A further example of a parameter of the penetration device and/or surrounding tissue of the anatomical structure is a thickness of tissue in the vicinity of the electrically-responsive elements of the penetration device. It is recognized that the thickness of tissue affects the dielectric properties of tissue. This, in turn, affects the electrical response of electrically-responsive elements in the vicinity of the tissue. It is therefore possible to correlate information about an electrical response of electrically responsive tissue to a thickness of tissue in the vicinity of the penetration device. As one example, a rate of change of an electrical response may be influenced by the thickness of nearby tissue, meaning that a thickness of nearby tissue can be derived by monitoring a rate of change of the electrical response. As another example, the electrical response may be directly correlated to a tissue thickness (e.g. based on predetermined calibration data).

The skilled person would be readily capable of identifying and determining other suitable examples of a parameter of the penetration device and/or surrounding tissue of the anatomical structure using the electrical responses of the electrically-responsive elements of the penetration device.

An understanding of the usefulness of obtaining one or more parameters of the penetration device and/or surrounding tissue of the anatomical structure using the electrical response(s) of the distal end of the needle, and two electrodes on the sheath (of which one is optionally an anchoring structure mounted on the sheath) will be hereafter described. In particular, tracking the location of these electrically-responsive elements of the penetration device (with respect to an anatomical model) provides useful information for performing a penetrating process.

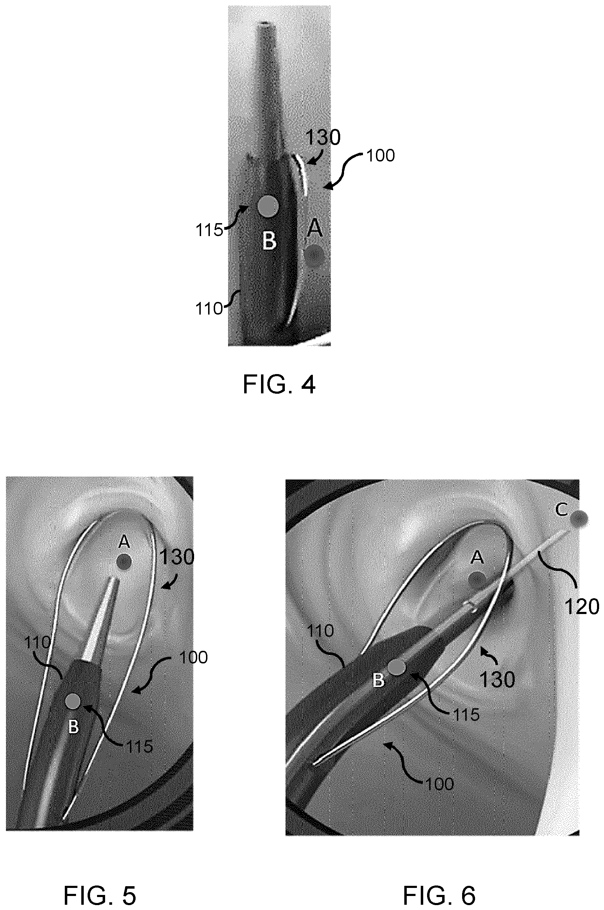

Figs. 4, 5 and 6 illustrate a penetration device 100 in various stages of a penetrating process, which are used to illustrate the usefulness of tracking the position/location of certain electrically-responsive elements of the penetration device throughout the penetrating process. In particular, by tracking the position of the three identified electrically-responsive elements of the penetration device, the full penetrating process can be readily monitored.

Initially, the penetration device may be configured as illustrated in Fig. 4, in which the needle (not visible) is covered by the sheath 120.

Point A represents the spatial location of the anchoring structure's 130 (here: a loop of metal) electrical weight center and will move according to predetermined mechanic properties of the penetration device. Point B represents the position of the first electrode 115 on the sheath.

As the penetration device moves in the anatomical structure (e.g. to reach a target location), the electrical response(s) of the first electrode 115 on the sheath and the (non-extended) anchoring structure 130 mounted on the sheath can be monitored, e.g. to track a location/orientation of the penetration device 100 and/or to perform an imaging process.

Thus, the electrical response of electrically-responsive elements of the penetration device can be used to derive one or more parameters of the penetration device and/or the anatomical structure, e.g. to generate an anatomical model of the anatomical structure.

The position of a desired anatomical feature or site for performing a penetrating process can be identified (e.g. from the generated anatomical model). For instance, for a transseptal procedure, the position of the fossa ovalis could be (automatically or manually) identified from the anatomical model.

After identifying a location at which a penetrating process is to be performed, the anchoring structure 130 may be extended out of the sheath 110 to brace or anchor the sheath against the tissue (in the vicinity of the anchoring structure). Since the mechanical behavior of the anchoring structure is known, a visual representation of the shape of the anchoring structure can also be displayed in real-time by monitoring the location of the anchoring structure 130.

This results in a penetration device being configured as illustrated in Fig. 5.

The sheath 110 can thereafter be rotated and deflected (by using the anchoring structure 130 as a brace) against the tissue) toward the desired anatomical site. The movement of the sheath can be monitored by tracking the location of the first electrode 115 on the sheath relative to the (now fixed) location of the anchoring structure 130. The movement of the sheath may cause the distal end of the sheath to displace or deform tissue of the anatomical structure (known as "tenting"). This displacement can be visually represented.

In particular, as the bounds of the anatomical structure are known (e.g. from the anatomical model) and the relationship between the first electrode 115 and the distal end of the sheath is known, it is possible to predict when the distal end of the sheath deforms the tissue of the anatomical structure (e.g. comes into contact with the bounds of the anatomical structure) by monitoring the location of the first electrode 115 with respect to the anatomical model.

The needle 120 can then be advanced through the sheath 110 (i.e. extend outwardly from a distal end of the sheath 110) to penetrate the tissue with which the distal end of the sheath makes contact, as illustrated in Fig. 6. The progress of the needle during the penetrating process can be tracked by monitoring the location of the distal end 125 of the needle, as indicated by point C of Fig. 6.

The tracked location of the distal end 125 of the needle may be used, together with the tracked location of the first electrode 115, to track an orientation and/or direction of the needle. This provides useful clinical information to the clinician for ensuring that the needle is correctly positioned in a desired penetration direction.

Of course, the electrical response of the needle may also be used before this final stage of the penetrating process. In particular, if the needle is near the distal end of the sheath (e.g. close, but not protruding from the sheath), then it will still respond to electric fields. This information can be used to improve a tracking of the penetration device during transport to the area for performing the penetration (e.g. to derive an orientation or shape of the penetration device), performing an imaging process (e.g. acting as another internal electrode for the imaging process) or to determine any other suitable parameter of the penetration device and/or surrounding tissue of the anatomical structure.

It has previously been explained how an anatomical model can be constructed using the response of internal electrodes to an electric field. This anatomical model can be rendered and displayed, e.g. at a user interface.

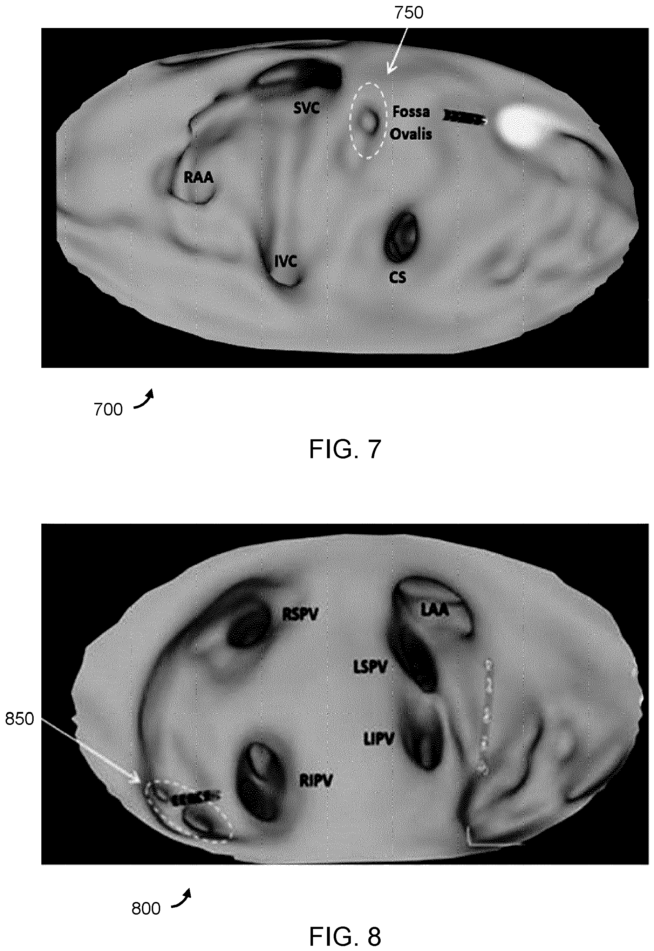

Fig. 7 and 8 respectively depict a first 700 and second 800 representation of a rendered anatomical model. A position 750 of the fossa ovalis is identifiable from the anatomical model. These figures also illustrate the position 850 of double transseptal tracts.

The figures also illustrate the position of a penetrating device, which is obtained by tracking the location and orientation of the penetrating device using a previously described approach, and providing a visual representation of the penetration device with respect to the anatomical model.

The advantages of the present invention, for improving an ease of performing a transsceptal procedure, are apparent from the representative images provided by Fig. 7 and 8. In particular, by providing a visual representation of the anatomy and the penetration device, the clinician is aided in the performance of a surgical procedure by their tracking and monitoring of the relative location of the penetration device.

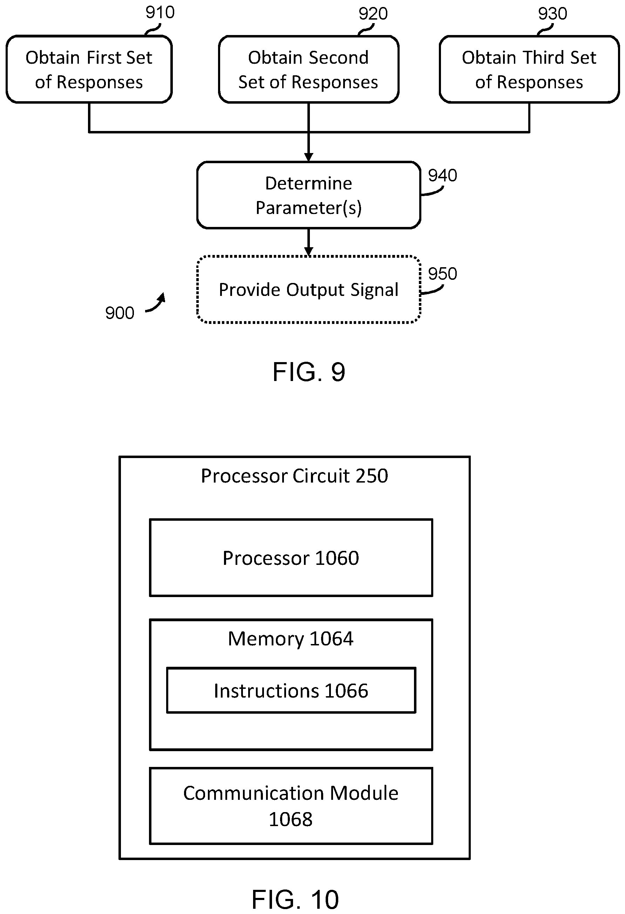

Fig. 9 is a flowchart illustrating a computer-implemented method 900 for determining one or more parameters of a penetration device, comprising a sheath that houses a needle having a distal end for penetrating tissue of an anatomical structure, and/or surrounding tissue of the anatomical structure.

The computer-implemented method comprises a step 910 of obtaining, from the distal end of the needle, a first set of one or more electrical responses of the distal end of the needle to one or more electrical fields induced in the anatomical structure, wherein the distal end of the needle is formed of a material responsive to changes in an electric field.

The computer-implemented method 900 comprises a step 920 of obtaining, from a first electrode mounted on the sheath, a second set of or more electrical responses of the electrode mounted on the sheath to one or more electrical fields induced in the anatomical structure.

The computed-implemented method also comprises a step 930 of obtaining, from a second electrode mounted on the sheath, a third set of one or more electrical responses of the second electrode to one or more electrical fields induced in the anatomical structure.

The computer-implemented method 900 comprises a step 9740 of determining one or more parameters of the penetration device and/or surrounding tissue of the anatomical structure using at least the first set of one or more electrical responses, the second set of one or more electrical responses and the third set of one or more electrical responses.

The method 900 may further comprise a step 950 of providing, to a further device, an output signal responsive to the determined one or more parameters of the penetration device and/or surrounding tissue of the anatomical structure.

The one or more parameters of the penetration device and/or surrounding tissue may be a relative position of the distal end of the needle and/or an orientation of the needle with respect to the anatomical structure.

Fig. 10 is a schematic diagram of a processor circuit 252, according to embodiments of the present disclosure. As shown, the processor circuit 1050 may include a (data) processor 1060, a memory 1064, and a communication module 1068. These elements may be in direct or indirect communication with each other, for example via one or more buses.

The processor 1060 may include a central processing unit (CPU), a digital signal processor (DSP), an ASIC, a controller, an FPGA, another hardware device, a firmware device, or any combination thereof configured to perform the operations described herein. The processor 1060 may also be implemented as a combination of computing devices, e.g., a combination of a DSP and a microprocessor, a plurality of microprocessors, one or more microprocessors in conjunction with a DSP core, or any other such configuration. In some embodiments, the processor is a distributed processing system, e.g. formed of a set of distributed processors.

The memory 1064 may include a cache memory (e.g., a cache memory of the processor 1060), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 1064 includes a non-transitory computer-readable medium. The non-transitory computer-readable medium may store instructions. For example, the memory 1064, or non-transitory computer-readable medium may have program code recorded thereon, the program code including instructions for causing the processor circuit 252, or one or more components of the processor circuit 252, particularly the processor 1060, to perform the operations described herein. For example, the processor circuit 252 can execute operations of the method 700. Instructions 1066 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 1064, with the code recorded thereon, may be referred to as a computer program product.

The communication module 1068 can include any electronic circuitry and/or logic circuitry to facilitate direct or indirect communication of data between the processor circuit 252, the penetration device and/or the user interface (or other further device). In that regard, the communication module 1068 can be an input/output (I/O) device. In some instances, the communication module 1068 facilitates direct or indirect communication between various elements of the processing circuit 252 and/or the system (Fig. 2).

In particular, the communication module 1068 may comprise the first input and the second input for obtaining the first and second inputs respectively. The communication module 1068 may also comprise an output for providing the output signal responsive to the determined one or more parameters of the penetration device and/or surrounding tissue of the anatomical structure.

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

The computer-readable program may execute entirely on a single computer/processor, partly on the computer/processor, as a stand-alone software package, partly on the computer/processor and partly on a remote computer or entirely on the remote computer or server (e.g. using a distributed processor processing system). In the latter scenario, the remote computer may be connected to the computer/processor through any type of network, including a local area network (LAN) or a wide area network (WAN), or the connection may be made to an external computer (for example, through the Internet using an Internet Service Provider).

It is also herein proposed to provide a processing system for determining one or more parameters of a penetration device, comprising a sheath that houses a needle having a distal end for penetrating tissue of an anatomical structure, and/or parameters of surrounding tissue of the anatomical structure, wherein the processing system comprises: an input interface configured to obtain: at a first input, and from an anchoring structure mounted on the sheath, a first set of one or more electrical responses of the anchoring structure mounted on the sheath to one or more electrical fields induced in the anatomical structure, wherein the anchoring structure is configured to secure or couple the sheath to tissue during a penetrating procedure of the penetration device; and a processor circuit configured to determine one or more parameters of the penetration device and/or surrounding tissue of the anatomical structure using at least the first set of one or more electrical responses.

Thus, it is also proposed to not necessary require the distal end of the needle to act as an electrode. Rather, information may be obtained by treating an anchoring structure used to brace the sheath against tissue as an electrode. Thus, embodiments of the invention recognize an advantage in treating an anchoring structure used for bracing the sheath against tissue as an electrode, such as reduced need for alternative electrodes and improved monitoring of a penetrating process.

This processing system may be adapted wherein the input interface is configured to obtain at a second input, and from an electrode mounted on the sheath, a second set of or more electrical responses of the electrode mounted on the sheath to one or more electrical fields induced in the anatomical structure, and the processor circuit is configured to determine one or more parameters of the penetration device and/or surrounding tissue of the anatomical structure further using at least the second set of one or more electrical responses.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A processing system (250) for determining one or more parameters of a penetration device (100), comprising a sheath (110) that houses a needle (120) having a distal end (125) for penetrating tissue of an anatomical structure, and/or parameters of surrounding tissue of the anatomical structure, the processing system comprises:
an input interface (251) configured to obtain (910, 920, 930):
at a first input (251A), and from the distal end of the needle, a first set of one or more electrical responses of the distal end of the needle to one or more electrical fields induced in the anatomical structure, wherein the distal end of the needle is formed of a material responsive to changes in an electric field;
at a second input (251B), and from a first electrode (115) mounted on the sheath, a second set of or more electrical responses of the first electrode to one or more electrical fields induced in the anatomical structure; and
at a third input (251C), and from a second electrode (130, 135) mounted on the sheath, a third set of one or more electrical responses of the second electrode to one or more electrical fields induced in the anatomical structure; and
a processor circuit (252) configured to determine (940) one or more parameters of the penetration device and/or surrounding tissue of the anatomical structure using at least the first set of one or more electrical responses, the second set of one or more electrical responses and the third set of one or more electrical responses.

2. The processing system of claim 1, further comprising an output interface configured to provide, to a further device, an output signal responsive to the determined one or more parameters of the penetration device and/or surrounding tissue of the anatomical structure.

3. The processing system of claim 1 or 2, wherein the processing system is further configured to display, at a user interface, a visual representation of the determined one or more parameters of the penetration device and/or surrounding tissue.

4. The processing system of any of claims 1 to 3, wherein the one or more parameters of the penetration device and/or surrounding tissue is a relative position of the sheath and/or the needle with respect to the anatomical structure.

5. The processing system of any of claims 1 to 4, wherein:
the input interface is further configured to obtain, at a fourth input and from an modelling system or memory, an anatomical model of the anatomical structure, and
wherein the one or more parameters of the penetration device and/or surrounding tissue comprises a relative location of the sheath and/or needle with respect to the anatomical model of the anatomical structure.

6. The processing system of claim 5, wherein the anatomical model of the anatomical structure is generated by monitoring the location of one or more electrodes mounted upon an intraluminal device.

7. The processing system of any of claims 1 to 6, wherein:
the first or second electrode comprises an anchoring structure configured to secure or couple the sheath to tissue during a penetrating procedure of the penetration device.

8. The processing system of claim 7, wherein the anchoring structure is moveable relative to other components of the sheath.

9. A medical system for penetrating tissue of a subject, the medical system comprising:
a penetration device comprising:
a needle having a distal end for penetrating tissue of an anatomical structure, the distal end of the needle being formed of a first material responsive to changes in an electric field;
a sheath that houses the needle;
an electrode mounted on the sheath; and
the processing system of any of claims 1 to 8.

10. The medical system of claim 9, wherein the distal end of the needle is configured to puncture the inter-atrial septum of a subject.

11. The medical system of any of claims 8 to 10, comprising one or more external electrodes, to be positioned externally to the subject, controllable to generate one or more electric fields, wherein the distal end of the needle is configured to be responsive to changes in the one or more electric fields generated by the one or more external electrodes.

12. A computer-implemented method (900) for determining one or more parameters of a penetration device (100), comprising a sheath (110) that houses a needle (120) having a distal end (125) for penetrating tissue of an anatomical structure, and/or surrounding tissue of the anatomical structure, the computer-implemented method comprising:
obtaining (910), from the distal end of the needle, a first set of one or more electrical responses of the distal end of the needle to one or more electrical fields induced in the anatomical structure, wherein the distal end of the needle is formed of a material responsive to changes in an electric field;
obtaining (920), from a first electrode (115) mounted on the sheath, a second set of or more electrical responses of the first electrode to one or more electrical fields induced in the anatomical structure;
obtaining (930), from a second electrode (130, 135) mounted on the sheath, a third set of one or more electrical responses of the second electrode to one or more electrical fields induced in the anatomical structure; and
determining (940) one or more parameters of the penetration device and/or surrounding tissue of the anatomical structure using at least the first set of one or more electrical responses and the second set of one or more electrical responses.

13. The computer-implemented method of claim 12, further comprising providing (950), to a further device, an output signal responsive to the determined one or more parameters of the penetration device and/or surrounding tissue of the anatomical structure.

14. The computer-implemented method of claim 13, wherein the one or more parameters of the penetration device and/or surrounding tissue is a relative position of the distal end of the needle with respect to the anatomical structure.

15. A computer program product comprising instructions which, when executed by a suitable computer or processing system, cause the computer to carry out the method of any of claims 12 to 14.
